# EUROPÄISCHE PATENTSCHRIFT

(11) **EP 0 867 192 B1**
(45) Veröffentlichungstag und Bekanntmachung des Hinweises auf die Patenterteilung: **18.05.2005**
(21) Anmeldenummer: 98102620.6
(22) Anmeldetag: 16.02.1998
(51) Int. Cl.: A61K 45/06, A61P 43/00

(54) **Kombinationspräparat zur Anwendung bei Demenz, enthaltend mindestens eine Verbindung die eine Acetylcholinesterase-Hemmwirkung oder muskarinerge Wirkung zeigt und eine Verbindung die den endogenen extrazellulären Adenosinspiegel erhöht**
Combinatory compositions for the treatment of dementia containing at least a compound having acetylcholinesterase-inhibitory or muscarinic activity and a compound that enhances the endogenous extracellular adenosine levels
Compositions combinées pour le traitement de la démence contenant au moins un produit à effet inhibiteur de acetylcholinestérase ou muscarinique et un produit qui accroit le niveau d'adénosine endogène extracellular

(30) Priorität: 26.02.1997 DE 19707655
(43) Veröffentlichungstag der Anmeldung: 30.09.1998
(73) Patentinhaber: Aventis Pharma Deutschland GmbH, 65929 Frankfurt am Main (DE)
(72) Erfinder: Schubert, Hans-Peter, Dr., 86974 Apfeldorf (DE); Nimmesgern, Hildegard, Dr., 64297 Darmstadt (DE); Rudolphi, Karl, Dr., 55116 Mainz (DE)

(56) Entgegenhaltungen:
- EP-A- 0 239 445
- EP-A- 0 260 127
- EP-A- 0 296 560
- WO-A-89/02739
- WO-A-91/04032
- WO-A-94/09787
- US-A- 4 719 212

## Beschreibung

Die Erfindung betrifft pharmazeutische Kombinationspräparate zur Behandlung der Demenz aufgrund neurodegenerativen Erkrankungen, die mit Untergang cholinerger Neurone und cholinergem Defizit einhergehen (Tohgi et al., Neurosci. Lett. 177 (1994), Seiten 1939-1942). Diese Kombinationspräparate kompensieren das cholinerge Defizit durch eine Verstärkung der über muskarinische Rezeptorstimulation ausgelösten Ca²⁺-abhängigen Signaltransduktion. Eine solche Verstärkung kann offenbar durch kooperative Adenosinwirkungen erreicht werden, und zwar durch eine Kombination von Substanzen, die die extrazelluläre Adenosinkonzentration erhöhen mit muskarinischen Rezeptoragonisten oder Acetylcholinesterasehemmem (ACHE-Hemmem).
Die bisher hauptsächlich verfolgte pharmakologische Strategie für die Therapie von Altersdemenzen ist die Aufrechterhaltung muskarinischer Rezeptoraktivierung durch Gabe von muskarinergen Agonisten oder von ACHE-Hemmern, die die Konzentration des endogenen Acetylcholins (ACH) am Rezeptor erhöhen. Ob bei fortschreitender Zerstörung cholinerger Neuronensysteme eine für eine regelrechte Zellfunktion ausreichende Erhöhung des ACH Spiegels durch ACHE Hemmung tatsächlich erreicht werden kann, ist fraglich. Ferner zeigen ACHE Hemmer bei mangelnder Spezifität erhebliche Nebenwirkungen. Eine Potenzierung der über muskarinische Rezeptoren vermittelten Wirkung insuffizienter ACH Konzentrationen durch andere Mechanismen wäre daher wünschenswert und könnte Grundlage sein für die Entwicklung einer entsprechenden Kombinationstherapie.

Es ist bekannt, daß ACH nicht nur die Funktionen von Nervenzellen, sondern auch von Gliazellen (Astrozyten) beeinflußt (Messamore et al., Neuroreport, 5 (1994), Seiten 1473-1476). Da pathologische Gliazellreaktionen in der Pathophysiologie der Demenz offenbar eine wichtige Rolle spielen (Akiyama et al., Brain Res. 632 (1993), Seiten 249-259), wurden kultivierte Astrozyten als in vitro Modellsystem gewählt. Die Beeinflussung der über muskarinische Rezeptoren ausgelösten intrazellulären Ca²⁺ Freisetzung wurde mit Hilfe der dynamischen Fluoreszenz-Imaging Methode untersucht.

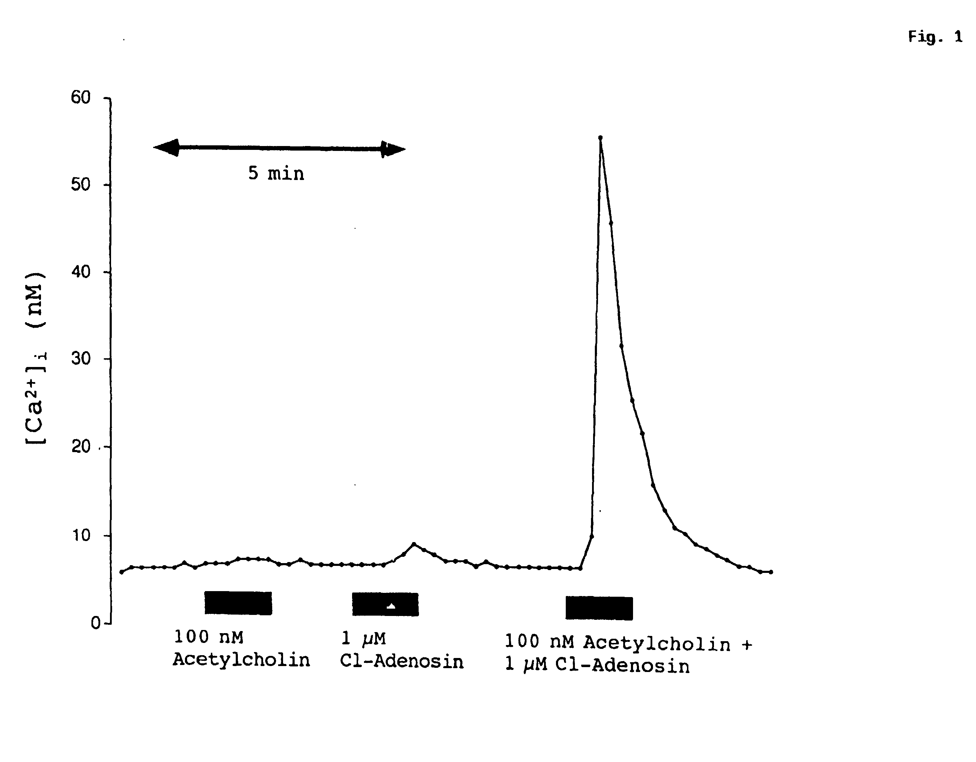

Es wurde nun gefunden, daß CI-Adenosin die muskarinerge intrazelluläre Ca²⁺ Freisetzung in kultivierten Astrozyten der Ratte konzentrationsabhängig potenziert (siehe Fig. 1 bis 3; Tabellen 1 und 2). So wird bereits in Gegenwart von 1 µM Cl-Adenosin eine etwa dreißigfache Potenzierung der durch ACH ausgelösten intrazellulären Ca²⁺ Erhöhung gemessen. Dieser Effekt war durch einen nikotinischen ACH-Rezeptorantagonisten nicht hemmbar, konnte aber durch einen muskarinischen Rezeptorantagonisten blockiert werden (Tabelle 3). Ebenso potenzierte CI-Adenosin die durch den muskarinergen Agonisten Oxotremorin-M ausgelöste intrazelluläre Ca²⁺ Freisetzung (Tabelle 4). Ein Großteil der den potenzierenden Cl-Adenosineffekt nachweisenden Experimente (n > 200) wird bei einer ACH Konzentration von 100 nM durchgeführt. In dieser niedrigen Konzentration ist ACH allein unwirksam. Auch CI-Adenosin allein ist über den getesteten Konzentrationsbereich (3 nM bis 3 µM) unwirksam. Dosis Wirkungsexperimente zur Bestimmung der für die Auslösung eines Ca²⁺ Signals in Kooperation mit 100 nM ACH notwendigen CI-Adenosinkonzentration ergeben einen potenzierenden Effekt bei mikromolaren CI-Adenosinkonzentrationen. Die Schwellenkonzentration liegt bei 1 µM. Da Cl-Adenosin in seiner Rezeptoraffinität dem endogenen Adenosin entspricht (Daly et al., Life Sci., 28 (1981), Seiten 2083-2097), heißt das, daß entsprechend auch eine Erhöhung des extrazellulären Adenosinspiegels vom physiologischen nanomolaren Konzentrationsbereich (Ballarin et al., Acta Physiol. Scand., 142 (1991), Seiten 97-103) auf 1 µM ausreicht, um unterschwellige ACH Konzentrationen am muskarinischen Rezeptor zur Wirkung zu bringen.

Aus diesen experimentellen Ergebnissen ergibt sich, daß die Beeinträchtigung der über muskarinische Rezeptoren vermittelten cholinergen Funktion bei Demenz durch eine Erhöhung der extrazellulären Adenosinkonzentration verbessert werden kann. Letzteres dürfte durch gekoppelte Applikation eines Adenosinaufnahmehemmers wie Propentofyllin möglich sein (Parkinson et al., Gem. Pharmacol. 25 (1994), Seiten 1053-1058). Eine pharmakologische Erhöhung der Extrazellulären Adenosinkonzentration würde auch eine geringere Dosierung des in einer Kombinationstherapie gegebenenfalls verwendeteten ACHE-Hemmers bzw. muskarinischen Rezeptoragonisten erlauben, was die Gefahr von unerwünschten Nebenwirkungen mindern würde.

Die Erfindung betrifft daher ein Kombinationspräparat, enthaltend mindestens
1) eine Verbindung, die eine Acetylcholinesterase-Hemmwirkung aufweist oder muskarinerge Wirkung zeigt ausgewählt aus der Gruppe Tetrahydroaminoacridin, 1-Benzyl-4-[(5,6-Dimethoxy-1-Indanon)-2-yl]-Methyl Piperidin und Milamelin,
2) eine Verbindung, die den endogenen extrazellulären Adenosinspiegel erhöht ausgewählt aus der Gruppe der Xanthinderivate der Formel 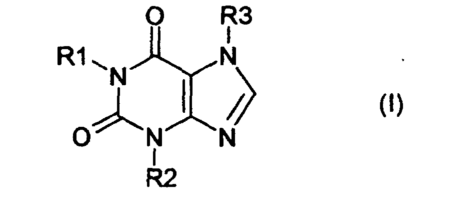 und/oder physiologisch verträgliche Salze der Verbindung der Formel I, wobei
   R¹ für
   a) Oxoalkyl mit 3 bis 8 Kohlenstoffatomen, deren Kohlenstoffkette geradkettig oder verzweigt sein kann, oder
   b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, steht,
   R² für
   a) Wasserstoffatom oder
   b) Alkyl mit 1 bis 4 Kohlenstoffatomen, deren Kohlenstoffkette geradkettig oder verzweigt sein kann, steht,
   R³ für
   a) Wasserstoffatom,
   b) Alkyl mit 1 bis 6 Kohlenstoffatomen, deren Kohlenstoffkette geradkettig oder verzweigt sein kann,
   c) Alkyl mit 1 bis 6 Kohlenstoffatomen, deren Kohlenstoffkette mit einem Sauerstoffatom unterbrochen ist, oder
   d) Oxoalkyl mit 3 bis 8 Kohlenstoffatomen, deren Kohlenstoffkette geradkettig oder verzweigt sein kann, steht, und
3) einen pharmazeutischen Träger,
   mit überadditiver Steigerung der muskarinischen Wirkung bei neurodegenerativen Erkrankungen zur gleichzeitigen, getrennten oder zeitlich abgestuften Anwendung.

Besonders bevorzugt wird 1-(5-Oxohexyl)-3-methyl-7-n-propyl-xanthin (Propentopfyllin) verwendet.

Geeignete physiologisch verträgliche Salze der Xanthinderivate der Formel I sind beispielsweise Alkali-, Erdalkali- oder Ammoniumsalze, einschließlich solcher von physiologisch verträglichen organischen Ammoniumbasen.

Die Herstellung der Verbindung der Formel I erfolgt unter Standardbedingungen auf bekannte Weise (US 4,289,776, US 4,833,146, US 3,737,433).

Die Ausgangsstoffe der Umsetzungen sind bekannt oder lassen sich nach literaturbekannten Methoden leicht herstellen. Unter dem Begriff "überadditiv" werden Wirkungen verstanden, die größer als die Summe der Einzelwirkungen sind.

Bevorzugte Kombinationspräparate enthalten Propentofyllin und 1-Benzyl-4-[(5,6-Dimethoxy-1-Indanon)-2-yl]-Methyl Piperidin.

Das erfindungsgemäße Kombinationspräparat eignet sich beispielsweise zur Behandlung von Demenz, insbesondere Altersdemenz.

Das erfindungsgemäße Kombinationspräparat kann auch Kombinationspackungen oder Kompositionen umfassen, in denen die Bestandteile nebeneinandergestellt sind und deshalb gleichzeitig, getrennt oder zeitlich abgestuft an ein und demselben menschlichen oder tierischen Körper angewendet werden können.

Das erfindungsgemäße Kombinationspräparat kann als Dosiereinheit in Form von Arzneiformen wie Kapseln (einschließlich Mikrokapseln, die im allgemeinen keine pharmazeutischen Träger enthalten), Tabletten (einschließlich Dragees und Pillen) oder Zäpfchen vorliegen, wobei bei Verwendung von Kapseln das Kapselmaterial die Funktion des Trägers wahrnehmen und der Inhalt z.B. als Pulver, Gel, Emulsion, Dispersion oder Lösung vorliegen kann. Besonders vorteilhaft und einfach ist es jedoch, orale (perorale) Formulierungen mit den beiden Wirkstoffkomponenten 1) und 2) herzustellen, die die berechneten Mengen der Wirkstoffe zusammen mit jedem gewünschten pharmazeutischen Träger enthalten. Auch eine entsprechende Formulierung (Zäpfchen) für die rektale Therapie kann angewandt werden. Ebenso ist die transdermale Applikation in Form von Salben oder Cremes, parenterale (intraperitoneale, subkutane, intravenöse, intraarterielle, intramuskuläre) Injektion oder Infusion von Lösungen oder orale Applikation von Lösungen, die die erfindungsgemäßen Kombinationen enthalten, möglich. Salben, Pasten, Cremes und Puder können neben den Wirkstoffen die üblichen Trägerstoffe enthalten, z.B. tierische und pflanzliche Fette, Wachse, Paraffine, Stärke, Traganth, Zellulosederivate, Polyethylenglykole, Silicone, Bentonite, Talkum, Zinkoxid, Milchzucker, Kieselsäure, Aluminiumhydroxid, Kalziumsilikat und Polyamidpulver oder Gemische dieser Stoffe.

Die Tabletten, Pillen oder Granulatkörper können nach üblichen Verfahren wie Preß-, Tauch- oder Wirbelbettverfahren oder Kesseldragierung hergestellt werden und enthalten Trägermittel und andere übliche Hilfsstoffe wie Gelatine, Agarose, Stärke (z.B. Kartoffel-, Mais- oder Weizenstärke), Zellulose wie Ethylzellulose, Siliziumdioxid, verschiedene Zucker wie Milchzucker, Magnesiumcarbonat und/oder Kalziumphosphate. Die Dragierlösung besteht gewöhnlich aus Zucker und/oder Stärkesirup und enthält meistens noch Gelatine, Gummi arabicum, Polyvinylpyrrolidon, synthetische Zelluloseester, oberflächenaktive Substanzen, Weichmacher, Pigmente und ähnliche Zusätze entsprechend dem Stand der Technik. Zur Herstellung der Arzneiformen kann jedes übliche Fließregulierungs-, Schmier- oder Gleitmittel wie Magnesiumstearat und Trennmittel verwendet werden.

Bevorzugt haben die Zubereitungen die Form von Mantel-/Kern- Tabletten oder Mehrschichttabletten, wobei sich die Wirkkomponente 2 im Mantel bzw. im Kern bzw. in einer Schicht befindet, während sich die Wirkkomponente 1 im Kern bzw. im Mantel bzw. in einer anderen Schicht befindet. Die Wirkstoffkomponenten können auch in retadierter Form vorliegen oder an Retadierungsmaterial adsorbiert bzw. im Retadierungsmaterial (z.B. solches auf Zellulose- oder Polystyrolharzbasis, z.B. Hydroxyethylzellulose) eingeschlossen sein. Eine verzögerte Freisetzung der Wirkstoffe kann auch erreicht werden, indem die betreffende Schicht bzw. das Kompartiment mit üblichen magensaftunlöslichen Überzügen versehen wird.

Die anzuwendende Dosierung ist selbstverständlich abhängig von verschiedenen Faktoren wie dem zu behandelnden Lebewesen (d.h. Mensch oder Tier), Alter, Gewicht, allgemeiner Gesundheitszustand, dem Schweregrad der Symptome, der zu behandelnden Erkrankung, eventuellen Begleiterkrankungen (falls vorhanden), der Art der begleitenden Behandlung mit anderen Arzneimitteln, oder der Häufigkeit der Behandlung. Die Dosierungen werden im allgemeinen mehrfach pro Tag und vorzugsweise einmal bis dreimal pro Tag verabreicht. Die verwendeten Mengen an Einzelwirkstoff orientieren sich hierbei an der empfohlenen Tagesdosis des jeweiligen Einzelwirkstoffs und sollen im allgemeinen im Kombinationspräparat von 10 % bis 100 % der empfohlenen Tagesdosis liegen, bevorzugt von 20 % bis 80 %, insbesondere bei 50 %. Die geeignete Therapie mit den erfindungsgemäßen Kombinationen besteht somit z.B. in der Verabreichung von einer Einzeldosierung der erfindungsgemäßen Kombinationspräparate bestehend aus
1) 100 mg bis 600 mg, vorzugsweise von 200 mg bis 400 mg Propentofyllin, insbesondere 300 mg Propentofyllin und
2) 2 mg bis 20 mg, vorzugsweise 5 mg bis 10 mg 1-Benzyl-4-[(5,6-Dimethoxy-1-Indanon)-2-yl]-Methyl Piperidin,
wobei die Menge naturgemäß von der Zahl der Einzeldosierungen und auch der zu behandelnden Krankheit abhängig ist und eine Einzeldosierung auch aus mehreren, gleichzeitig verabreichten Dosiereinheiten bestehen kann.

### Pharmakologische Beispiele

### Astrozytenkulturen

Die Cortex-Astrozytenkulturen stammen von der Hirnrinde 19-20 Tage alter Wistar-Ratten Embryos, die dem Muttertier nach Töten unter Äthernarkose entnommen wurden. Nach Präparation des Gehirns wurde Rindengewebe mit einer Pipette angesaugt und in Dulbecco's modifiziertem Eagle-Medium (DEM) unter Zusatz von 15% fetalem Kälberserum aufgenommen. Nach Filtrieren durch Linsenpapier wurde die Zellsuspension auf (mit Polyethylenimin-beschichteten) Glas-Objekträgern ausgepflanzt und in Kulturflaschen bei zweimaligem Mediumwechsel pro Woche unter Standardbedingungen im Brutschrank gezüchtet. Nach etwa 7 Tagen wurden die Zellen geerntet und nach Trypsinierung (um Nervenzellen zu eleminieren) wieder ausgepflanzt in einer Konzentration von 5 x 10⁴ Zellen/cm² und für weitere 6-8 Tage bis Versuchsbeginn gezüchtet. Diese Kulturen bestanden zu mehr als 95% aus Astrozyten, die eine positive Immunreaktion für den Astrozytenmarker GFAP (saures Glia fibrilläres Protein) aufwiesen.

Fluoreszenz-Imaging Experimente zur Messung der intrazellulären Ca²⁺ Konzentration und ihrer experimentellen Beeinflussung.
Zu Beginn des Versuchs (6-8 Tage nach dem Wiederauspflanzen) wurden die kultivierten Astrozyten mit einem Ca²⁺-Fluoreszenzmarker aufgeladen, und zwar durch Inkubation mit 5 µM Fura-2-Acetoxymethyl-Ester (Molecular Probes) in BHKR (bikabonisierter, Hepes-gepufferter Krebs-Ringerlösung) bei 37°C für 1 Stunde. Nach dem Aufladen wurden die die kultivierten Astrozyten enthaltenden Glasträger in eine Meßkammer übertragen und auf dem zum Fluoreszenz-Imaging-Meßplatz gehörigen Invert-Fluoreszenzmikroskop (Zeiss Axiovert 100, Zeiss Fluar 40 x Objektiv) installliert. Hier wurde die Kammer während der Versuchsdauer (in der Regel 20-30 Min.) kontinuierlich mit Temperatur-kontrolliertem (37°C) BHKR perfundiert bei einer Flußrate von 600 µl/min. Die Messung erfolgte mit dem FUCAL-Fluoreszenz-Imaging-System (T.I.L.L Photonics GmbH, Planegg), wobei nach Anregung durch zwei Exzitationswellenlängen bei 340 und 380 nm die emittierte Fluoreszenz oberhalb der Wellenlänge von 420 nm mit Hilfe einer CCD Kamera (CS 90, Theta System, Gröbenzell) gemessen und die dementsprechende Ca²⁺ Konzentration errechnet wurde. Die Messungen erfolgten in Zeitabständen von 12 Sek. jeweils vor, während und nach Zugabe der verschiedenen Testsubstanzen zum perfundierten Medium. Änderungen der intrazellulären Ca²⁺ Konzentration wurden auf Einzelzellebene bestimmt und zwar in verschiedenen, jeweils adäquat festgelegten Meßfenstern.
Die verschiedenen Testsubstanzen (Acetylcholin oder Oxotremorin in Gegenwart oder Abwesenheit von CI-Adenosin) wurden in der Regel für die Zeitdauer von 1 Minute dem Perfusionsmedium zugegeben. Da die ausgelösten intrazellulären Ca²⁺ Anstiege transient waren, wurden die in den Ergebnis-Tabellen und -Kurven ausgewiesenen Änderungen der intrazellulären Ca²⁺ Konzentrationen auf die jeweils gemessenen Gipfelwerte bezogen.

**Tabelle 1**

| Erhöhung der intrazellulären Ca²⁺- Konzentration (nM) | | |
|---|---|---|
| Acetylcholin (nM) | intrazelluläre Ca²⁺-Konzentration [nM] | |
| | ohne Zusatz | + 1µM Cl-Adenosin |
| 3 | | 14.17 ± 2.87 |
| 10 | | 23.59 ± 4.25 |
| 100 | 2.18 ± 1.8 | 56.27 ± 6.7 |
| 300 | 1.4 ± 1.57 | 107.27 ± 9.41 |
| 1 000 | 19.83 ± 3.24 | |
| 3 000 | 41.91 ± 5.59 | 182.18 ± 12.27 |
| 6 000 | 73.79 ± 10.94 | |
| 10 000 | 126.79 ± 12.07 | 220.99 ± 10.97 |
| 30 000 | | 214.20 ± 10.38 |
| 100 000 | 192.74 ± 16.61 | |
| 300 000 | 203.49 ± 15.46 | |
| Durchschnittswerte ±SEM, Anzahl der gemessenen Zellen n=45 (für jeden Meßwert) | | |

Tabelle 1 zeigt, daß die Dosis-Wirkungskurve der durch ACH in Astrozyten ausgelösten intrazellulären Ca²⁺ Erhöhung bei gleichzeitiger Wirkung von 1 µM Cl-Adenosin erheblich nach links verschoben wird. Hier muß der ACH Spiegel lediglich 100 nM betragen, um ein gleich großes Ca²⁺ Signal zu erzeugen, für das eine dreißigfach höhere ACH Konzentration (mehr als 3 µM) notwendig wäre in Abwesenheit einer kooperierenden Adenosinwirkung. Die für diesen kooperativen Effekt benötigte kritische Adenosinerhöhung liegt in einem Bereich, der pharmakologisch durch Therapie mit dem Adenosin-Aufnahme Blocker Propentofyllin erreicht werden sollte.

**Tabelle 2**

| Cl-Adenosin (nM) | intrazelluläre Ca²⁺- Konzentration [nM] | |
|---|---|---|
| | ohne Zusatz | + 100 nM Acetylcholin |
| 3 | 10.54 ± 1.57 | 1.57 ± 2 |
| 30 | 8.29 ± 1.69 | 5.06 ± 3.28 |
| 100 | 11.31 ± 1.92 | 4.82 ± 3.43 |
| 300 | 8.45 ± 1.64 | 11.56 ± 4.42 |
| 1 000 | 14.2 ± 2.23 | 32.35 ± 5.49 |
| 3 000 | 15.99 ± 2.26 | 104.16 ± 13.82 |
| Durchschnittswerte ±SEM, Anzahl der gemessenen Zellen n=40 (für jeden Meßwert) | | |

Tabelle 2 zeigt für eine Ca²⁺ Mobilisation notwendigen Cl-Adenosinkonzentrationen in Gegenwart von 100 nM ACH.

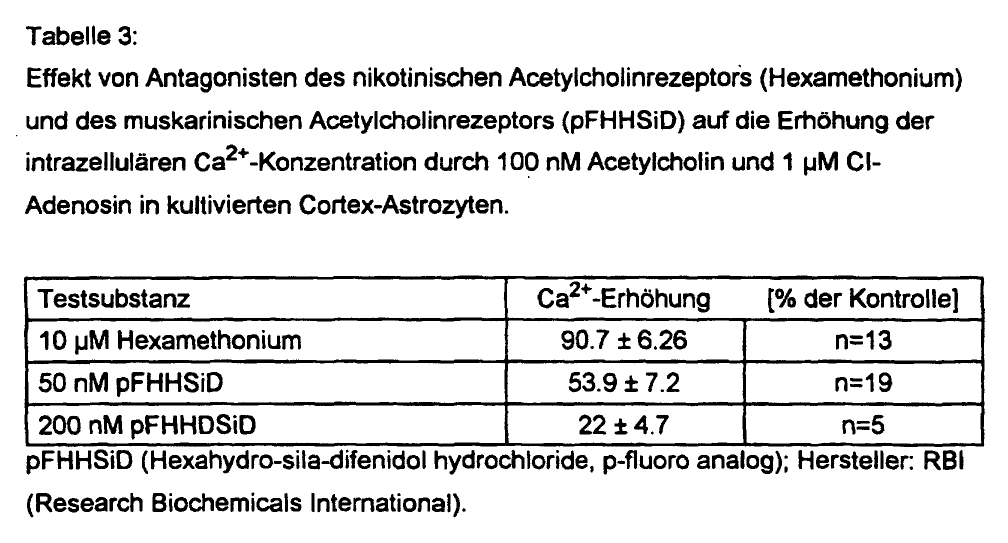

Durchschnittswerte ±SEM in Prozent der intrazellulären Ca²⁺-Erhöhung, die in Abwesenheit der Antagonisten durch 100 nM Acetylcholin und 1 µM Cl-Adenosin erzielt wurde (Kontrollwert = 100%). Als Kontrollwert wurde eine intrazelluläre Ca²⁺-Erhöhung von 98.4 6.4 nM gemessen (n=125).

Tabelle 3 zeigt, daß der durch Cl-Adenosin potenzierte ACH-Effekt einen repräsentativen, über muskarinische Rezeptoren vermittelten ACH-Effekt darstellt, der durch einen muskarinischen Rezeptorblocker antagonisiert wird, nicht aber durch einen nikotinischen Rezeptorblocker.

**Tabelle 4**

| Effekt von CI-Adenosin und dem muskarinischen Acetylcholinrezeptor-Agonisten Oxotremorin-M auf den intrazellulären Ca²⁺-Gehalt in kultivierten Cortex-Astrozyten. | |
|---|---|
| Acetylcholinrezeptor Agonist | Ca²⁺-Erhöhung [nM] |
| 100 nM Oxotremorin-M | 3,3 ± 5,7 |
| 100 nM Oxotremorin + 1 µM Cl-Adenosin | 93,3 ± 23,7 |
| Durchschnitswerte ±SEM (n=6). | |

Tabelle 4 zeigt, daß Cl-Adenosin auch das durch einen muskarinischen Rezeptoragonisten ausgelöste Ca²⁺ Signal potenziert.

Fig. 1 zeigt die Ergebnisse eines Fluoreszenz-Imaging Experiment wie auf Seite 8 beschrieben. 100 nM ACH sowie 1 µM Cl-Adenosin alleine eingesetzt sind unwirksam. Ihre Kombination führt zu einer dramatischen Erhöhung der intrazellulären Ca²⁺-Konzentration innerhalb der Astrozyten. Die in Ca²⁺ freiem Medium durchgeführte Experimente zeigen dagegen einen geringeren, aber immer noch intrazellulären massiven Ca²⁺ Anstieg innerhalb der Astrozyten wenn ACH und Cl-Adenosin zusammen eingesetzt werden; dies zeigt, daß intrazelluläres Ca²⁺ mobilisiert wird.

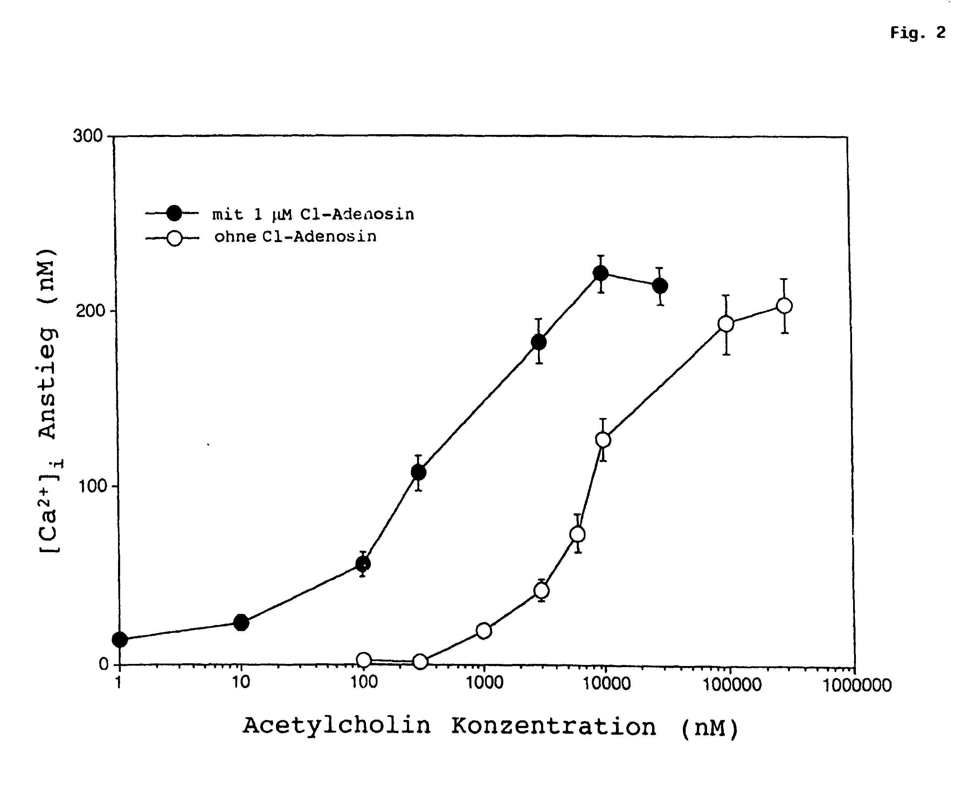

Fig. 2 zeigt ein Fluoreszenz-Imaging Experiment wie auf Seite 8 beschrieben, wobei die Dosis-Wirkungskurve der durch ACH in Astrozyten ausgelösten intrazellulären Ca²⁺ Erhöhung bei gleichzeitiger Wirkung von 1 µM Cl-Adenosin erheblich nach links verschoben wird. Hier muß der ACH Spiegel lediglich 100 nM betragen, um ein gleich großes Ca²⁺ Signal zu erzeugen, für das eine dreißigfach höhere ACH Konzentration (mehr als 3 µM) notwendig wäre in Abwesenheit einer kooperierenden Adenosinwirkung.

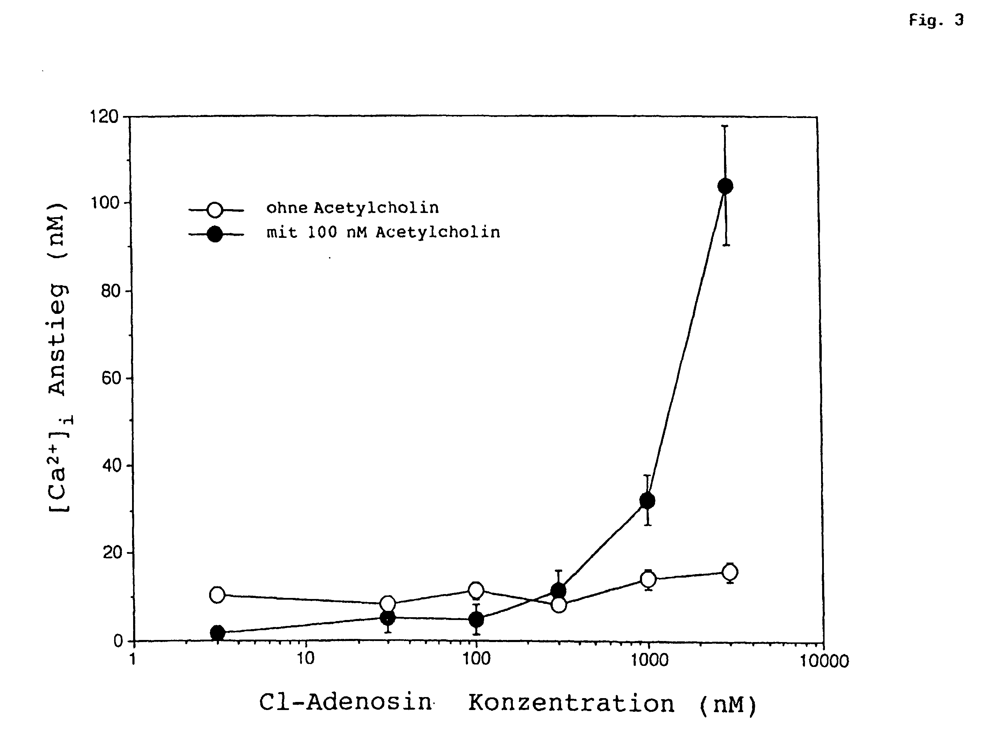

Fig. 3 zeigt die Ergebnisse eines Fluoreszenz-Imaging Experiments wie beschrieben auf Seite 8, wobei die für eine Ca²⁺ Mobilisation notwendigen Cl-Adenosinkonzentrationen in Gegenwart von 100 nM ACH bestimmt wird.

## Patentansprüche

1. Kombinationspräparat, enthaltend mindestens
1) eine Verbindung, die eine Acetylcholinesterase-Hemmwirkung aufweist oder muskarinerge Wirkung zeigt ausgewählt aus der Gruppe Tetrahydroaminoacridin, 1-Benzyl-4-[(5,6-Dimethoxy-1-Indanon)-2-yl]-Methyl Piperidin und Milamelin,
2) eine Verbindung, die den endogenen extrazellulären Adenosinspiegel erhöht ausgewählt aus der Gruppe der Xanthinderivate der Formel 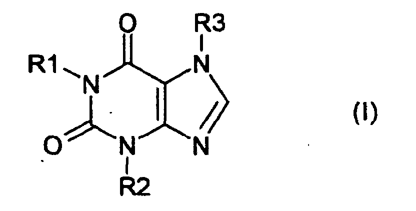 und/oder physiologisch verträgliche Salze der Verbindung der Formel I, wobei
R¹ für
a) Oxoalkyl mit 3 bis 8 Kohlenstoffatomen, deren Kohlenstoffkette geradkettig oder verzweigt sein kann, oder
b) Hydroxyalkyl mit 1 bis 8 Kohlenstoffatomen, deren Kohlenstoffkette geradkettig oder verzweigt sein kann und deren Hydroxygruppe eine primäre, sekundäre oder tertiäre Alkoholfunktion darstellt, steht,
R² für
a) Wasserstoffatom oder
b) Alkyl mit 1 bis 4 Kohlenstoffatomen, deren Kohlenstoffkette geradkettig oder verzweigt sein kann, steht,
R³ für
a) Wasserstoffatom,
b) Alkyl mit 1 bis 6 Kohlenstoffatomen, deren Kohlenstoffkette geradkettig oder verzweigt sein kann,
c) Alkyl mit 1 bis 6 Kohlenstoffatomen, deren Kohlenstoffkette mit einem Sauerstoffatom unterbrochen ist, oder
d) Oxoalkyl mit 3 bis 8 Kohlenstoffatomen, deren Kohlenstoffkette geradkettig oder verzweigt sein kann, steht, und
3) einen pharmazeutischen Träger,
mit überadditiver Steigerung der muskarinischen Wirkung bei neurodegenerativen Erkrankungen zur gleichzeitigen, getrennten oder zeitlich abgestuften Anwendung.

2. Kombinationspräparat gemäß Anspruch 1, **dadurch gekennzeichnet, daß** die darin enthaltene Verbindungen Propentopfyllin und 1-Benzyl-4-[(5,6-Dimethoxy-1-Indanon)-2-yl]-Methyl Piperidin sind.

3. Verwendung des Kombinationspräparat gemäß der Ansprüche 1 oder 2 zur Herstellung eines Arzneimittels zur Behandlung neurodegenerativer Erkrankungen, insbesondere Altersdemenz.

## Claims

1. A combination preparation, comprising at least
1) a compound which has an acetylcholinesterase-inhibitory action or exhibits muscarinergic action selected from the group consisting of tetrahydroaminoacridine, 1-benzyl-4-[(5,6-dimethoxy-1-indanon)-2-yl]methylpiperidine and milameline,
2) a compound which increases the endogenous extracellular adenosine level selected from the group consisting of the xanthine derivatives of the formula 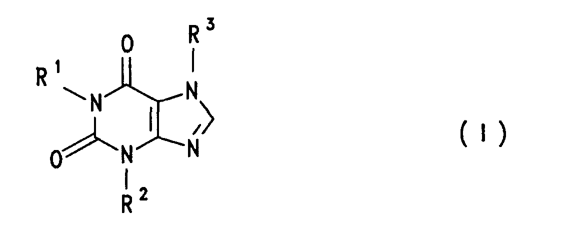 and/or physiologically tolerable salts of the compound of the formula I, where R¹ is
a) oxoalkyl having 3 to 8 carbon atoms, whose carbon chain can be straight-chain or branched, or
b) hydroxyalkyl having 1 to 8 carbon atoms, whose carbon chain can be straight-chain or branched and whose hydroxyl group is a primary, secondary or tertiary alcohol function,
R² is
a) a hydrogen atom or
b) alkyl having 1 to 4 carbon atoms, whose carbon chain can be straight-chain or branched,
R³ is
a) a hydrogen atom,
b) alkyl having 1 to 6 carbon atoms, whose carbon chain can be straight-chain or branched,
c) alkyl having 1 to 6 carbon atoms, whose carbon chain is interrupted by an oxygen atom, or
d) oxoalkyl having 3 to 8 carbon atoms, whose carbon chain can be straight-chain or branched, and
3) a pharmaceutical excipient,
having a superadditive increase in the muscarinic action in neurodegenerative disorders for simultaneous, separate or sequential administration.

2. A combination preparation as claimed in claim 1, wherein the compounds contained therein are propentopfylline and 1-benzyl-4-[(5,6-dimethoxy-1-indanon)-2-yl]methylpiperidine.

3. The use of the combination preparation as claimed in claims 1 or 2 for the production of a pharmaceutical for the treatment of neurodegenerative disorders, in particular senile dementia.

## Revendications

1. Préparation combinée contenant au moins
1) un composé présentant un effet d'inhibition de l'acétylcholinestérase ou un effet muscarinique, choisi dans le groupe constitué par la tétrahydroaminoacridine, la 1-benzyl-4-[(5,6-diméthoxyl-indanon)-2-yl]méthylpipéridine et la milaméline,
2) un composé qui augmente le niveau d'adénosine extracellulaire endogène, choisi dans le groupe constitué par les dérivés de la xanthine de formule 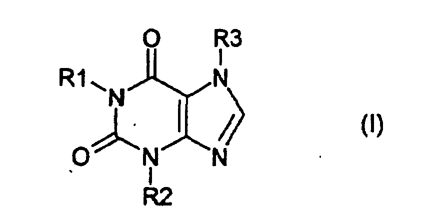 et/ou les sels physiologiquement acceptables du composé de formule I, où
R¹ représente
a) un groupe oxoalkyle comprenant 3 à 8 atomes de carbone, dont la chaîne carbonée peut être linéaire ou ramifiée, ou
b) un groupe hydroxyalkyle comprenant 1 à 8 atomes de carbone, dont la chaîne carbonée peut être linéaire ou ramifiée et dont le groupe hydroxy représente une fonction alcool primaire, secondaire ou tertiaire
R² représente
a) un atome d'hydrogène ou
b) un groupe alkyle comprenant 1 à 4 atomes de carbone, dont la chaîne carbonée peut être linéaire ou ramifiée,
R³ représente
a) un atome d'hydrogène ou
b) un groupe alkyle comprenant 1 à 6 atomes de carbone, dont la chaîne carbonée peut être linéaire ou ramifiée,
c) un groupe alkyle comprenant 1 à 6 atomes de carbone, dont la chaîne carbonée est interrompue par un atome d'oxygène, ou
d) un groupe oxoalkyle comprenant 3 à 8 atomes de carbone, dont la chaîne carbonée peut être linéaire ou ramifiée et
3) un support pharmaceutique,
avec une augmentation synergique de l'effet muscarinique lors de maladies neurodégénératives pour une utilisation simultanée, séparée ou échelonnée dans le temps.

2. Préparation combinée selon la revendication 1, **caractérisée en ce que** les composés qui y sont contenus sont la propentopfylline et la 1-benzyl-4-[(5,6-diméthoxy-1-indanon)-2-yl]méthylpipéridine.

3. Utilisation de la préparation combinée selon les revendications 1 ou 2 pour la préparation d'un médicament destiné au traitement de maladies neurodégénératives, en particulier la démence sénile.
